(19) Europäisches Patentamt
European Patent Office
Office européen des brevets

(11) Veröffentlichungsnummer : **0 003 562**
**B1**

(12) # EUROPÄISCHE PATENTSCHRIFT

(45) Veröffentlichungstag der Patentschrift : **08.07.81**

(51) Int. Cl.³ : **C 07 C 43/23**, C 07 C 79/35, C 07 C121/75, C 07 D247/00

(21) Anmeldenummer : **79100293.4**

(22) Anmeldetag : **01.02.79**

(54) **Verfahren zur Herstellung von Hydroxyphenyläthern.**

(30) Priorität : **13.02.78 DE 2805983**

(43) Veröffentlichungstag der Anmeldung : **22.08.79 (Patentblatt 79/17)**

(45) Bekanntmachung des Hinweises auf die Patenterteilung : **08.07.81 Patentblatt 81/27**

(84) Benannte Vertragsstaaten : **BE CH DE FR GB IT NL**

(56) Entgegenhaltungen :
**DE - A - 2 157 781**
**DE - A - 2 433 066**
**DE - B - 2 007 737**
**Chemical Abstracts vol. 48, no 12, 1954, Columbus, Ohio, USA**
**G. ILLUMINATI et al. " Reactivity of the heterocyclic nuclear halogen in the Friedel-Crafts reaction.**
**Preparation of some (dihydroxyphenyl) quinoline and -benzothiazole derivates ". Spatte 7011 a-e**

(73) Patentinhaber : **BAYER AG**
**Zentralbereich Patente, Marken und Lizenzen**
**D-5090 Leverkusen 1, Bayerwerk (DE)**

(72) Erfinder : **Förster, Heinz, Dr.**
**Am Eckbusch 47**
**D-5600 Wuppertal 1 (DE)**

**EP 0 003 562 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Verfahren zur Herstellung von Hydroxyphenyläthern

Die vorliegende Erfindung betrifft ein neues Verfahren zur Herstellung von teilweise bekannten Hydroxyphenyläthern, die als Zwischenprodukte zur Synthese von herbiziden Wirkstoffen verwendet werden können.

Es ist bereits bekannt geworden, daß sich Nitrohydroxydiphenyläther durch Umsetzung von p-Nitrochlorbenzol mit Alkalisalzen von Dihydroxybenzolen herstellen lassen (vgl. Deutsche Offenlegungsschrift 21 57 781). Dieses Verfahren weist jedoch eine Reihe von Nachteilen auf. So ist est auf die Herstellung von Diphenyläthern beschränkt. Ferner ist es nicht gleichermaßen gut zur Umsetzung der verschiedenen Dihydroxybenzole geeignet. Während aus den Alkalisalzen von para-Dihydroxybenzolen, wie Hydrochinon, und p-Nitrochlorbenzol die entsprechenden Nitrohydroxydiphenyläther in sehr guten Ausbeuten gebildet werden, erhält man beispielsweise beim Ausgehen von meta-Dihydroxyverbindungen und bei analoger Verfahrensführung die gewünschten Produkte nur in mäßigen Ausbeuten und minderer Qualität, was unter anderem auf die Bildung von Bis-äthern zurückzuführen ist.

Weiterhin ist bereits bekannt geworden, daß man durch Umsetzung von 2 Mol 3,4,5-Trichlorbenzotrifluorid mit 1 Mol Kaliumsalz des Resorcins das 1,3-Bis-(2,6-dichlor-4-trifluormethyl-phenoxy)-benzol erhält (vgl. DT-OS 23 11 628). Versucht man in analoger Weise den 2,6-Dichlor-4-trifluormethyl-3'-hydroxy-diphenyläther zu synthetisieren, so gelingt dies nur in unbefriedigender Weise. Zum einen entsteht beim Arbeiten bei etwa 140 °C ein sehr uneinheitliches Produkt, aus dem sich der gewünschte 2,6-Dichlor-4-trifluormethyl-3'-hydroxy-diphenyläther nur unter großen Schwierigkeiten in geringer Menge isolieren läßt. Zum anderen bildet sich beim Arbeiten bei einer Temperatur von etwa 160 °C eine beträchtliche Menge an 1,3-Bis-(2,6-dichlor-4-trifluormethyl-phenoxy)-benzol.

Es wurde nun gefunden, daß man die teilweise bekannten Hydroxyphenyläther der Formel

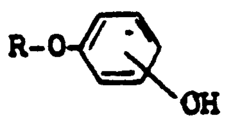

(I)

in welcher R für einen Rest der Formel

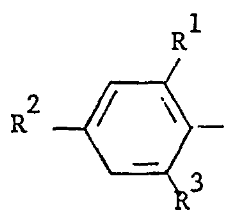

steht,

worin $R^1$, $R^2$ und $R^3$ gleichartig oder verschieden sind und für Wasserstoff, Chlor, Trifluormethyl, Nitro oder Cyano stehen, wobei jedoch mindestens einer der Reste $R^1$, $R^2$ und $R^3$ für Trifluormethyl, Nitro oder Cyano steht und mindestens einer der Reste $R^1$, $R^2$ und $R^3$ für Wasserstoff steht, und

R ferner für einen gegebenenfalls durch Chlor, Nitro und/oder Cyano substituierten 5- oder 6-gliedrigen heterocyclischen Rest steht, der 1-3 Heteroatome, wie Stickstoff, Schwefel und/oder Sauerstoff im Ring enthalten kann und außerdem mit einem gegebenenfalls durch Chlor und/oder Trifluormethyl substituierten Benzolring anelliert sein kann, erhält, wenn man Halogen-Verbindungen der Formel

R—Hal (II)

worin R die oben angegebene Bedeutung hat und Hal für Chlor oder Brom steht, mit Dihydroxybenzolen der Formel

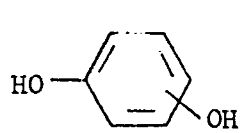

(III)

in Gegenwart von Calciumhydroxid sowie in Gegenwart eines polaren Verdünnungsmittel, bei Temperaturen zwischen 20 °C und 200 °C umsetzt.

Es ist als ausgesprochen überraschend zu bezeichnen, daß Hydroxyphenyläther der Formel (I) nach dem erfindungsgemäßen Verfahren in hoher Ausbeute und ausgezeichneter Reinheit zugänglich sind, denn im Hinblick auf den bekannten Stand der Technik war zu erwarten, daß bei dem Verfahren die gleichen Komplikationen auftreten wie bei der analogen Umsetzung von p-Nitrochlorbenzol mit Dihydroxybenzolen in Gegenwart von Alkalihydroxiden. Insbesondere war keineswegs vorauszusehen, daß sich Bildung an unerwünschten Bis-äthern durch einen Ersatz von Alkalihydroxiden durch die in der organischen Chemie kaum benutzte Base Calciumhydroxid weitgehend unterdrücken läßt.

Das erfindungsgemäße Verfahren weist eine Reihe von Vorteilen auf. So ist es nicht auf die Synthese von Diphenyläthern beschränkt, sondern läßt sich relativ breit anwenden.

Ganz abgesehen davon ist es auch im technischen Maßstab in verhältnismäßg einfacher Weise durchführbar. Die Hydroxyphenyläther fallen bei dem erfindungsgemäßen Verfahren, — wie bereits erwähnt —, in hoher Ausbeute und ausgezeichneter Reinheit nahezu frei von störenden Nebenprodukten an. Ein zusätzlicher Vorteil besteht darin, daß die Aufarbeitung keine Probleme bietet. Die Produkte werden nach dem Verdünnen und Ansäuren des Reaktionsgemisches meist in kristalliner Form erhalten und lassen sich ohne Schwierigkeiten abfiltrieren. Im übrigen ist das als Base eingesetzte Calciumhydroxid ein besonders preisgünstiges technisches Produkt. Das erfindungsgemäße Verfahren stellt somit eine wertvolle Bereicherung der Technik dar.

Verwendet man 3,4,5-Trichlor-benzotrifluorid und Resorcin als Ausgangsverbindungen, so kann der Reaktionsverlauf durch das folgende Formelschema wiedergegeben werden :

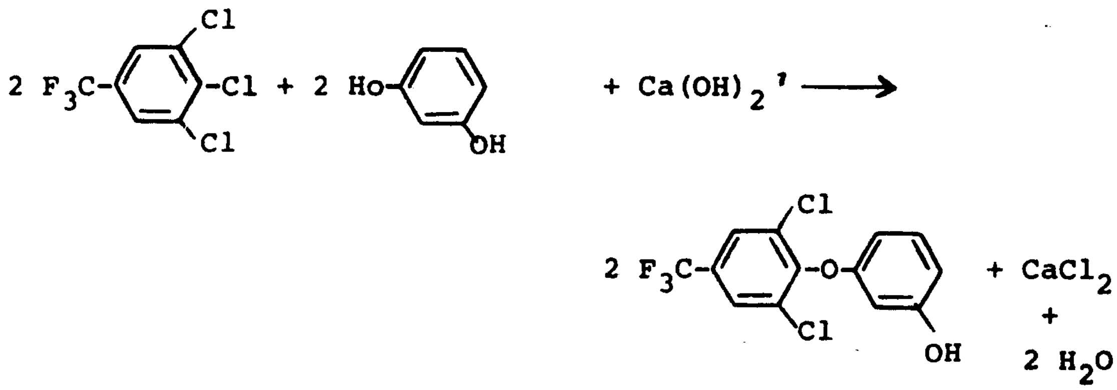

Die als Ausgangstoffe benötigten Halogenverbindungen sind durch die Formel (II) allgemein definiert.

Insbesondere in Frage kommende Heterocyclen sind hierbei solche, die sich durch die nachstehenden Formeln wiedergeben lassen :

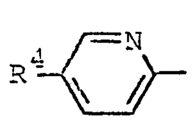 , 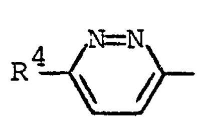 , 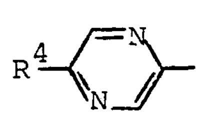 und 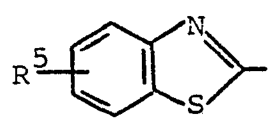

(IV) (V) (VI) (VII)

In den Formeln (IV) bis (VI) steht $R^4$ jeweils insbesondere für Wasserstoff, Chlor, Nitro oder Cyano. In der Formel (VII) steht $R^5$ insbesondere für Wasserstoff, Chlor oder Trifluormethyl.

Die erfindungsgemäß verwendbaren Ausgangsstoffe der Formel II sind bereits bekannt oder können nach bekannten Verfahren hergestellt werden. Als Beispiele seien genannt :

1-Chlor-2-trifluormethyl-, 1-Chlor-4-trifluormethyl-, 1-Chlor-2-nitro-, 1-Chlor-4-nitro-, 1-Chlor-2-cyano-, 1-Chlor-4-cyano-, 1,2-Dichlor-4-trifluormethyl-, 1,4-Dichlor-2-trifluormethyl-, 1,2-Dichlor-4-nitro-, 1,4-Dichlor-2-nitro-, 1,2-Dichlor-4-cyano-, 1,4-Dichlor-2-cyano-, 1,2,3-Trichlor-5-trifluormethyl-, 1,2,3-Trichlor-5-nitro-, 1,2,3-Trichlor-5-cyano-, 1-Chlor-2,4-bistrifluormethyl-, 1-Chlor-2,4-dinitro-, 1-Chlor-2,4-dicyano-, 1-Chlor-2-trifluormethyl-4-nitro-, 1-Chlor-4-trifluormethyl-2-nitro-, 1-Chlor-2-trifluormethyl-4-cyano-, 1-Chlor-4-trifluormethyl-2-cyano-, 1-Chlor-2-nitro-4-cyano- und 1-Chlor-4-nitro-2-cyano-benzol ;

2-Chlor-, 2,5-Dichlor-, 2-Chlor-5-nitro- und 2-Chlor-5-cyanopyridin ;

2-Chlor-, 2,5-Dichlor-, 2-Chlor-5-nitro- und 2-Chlor-5-cyanopyridazin ;

2-Chlor-, 2,5-Dichlor-, 2-Chlor-5-nitro- und 2-Chlor-5-cyanopyrazin ;

2-Chlor-, 2,4-, 2,5-, 2,6- und 2,7-Dichlor-, 4-, 5-, 6- und 7-Trifluormethyl-2-chlor-, benzthiazol.

Die weiterhin als Ausgangsstoffe benötigen Dihydroxybenzole sind durch die Formel (III) definiert. Vorzugsweise verwendbar sind Resorcin und Hydrochinon.

Als Base wird bei dem erfindungsgemäßen Verfahren Calciumhydroxid verwendet.

Als Verdünnungsmittel, die bei der Durchführung des erfindungsgemäßen Verfahrens eingesetzt werden können, kommen alle polaren aprotischen Lösungsmittel in Betracht. Hierzu gehören vorzugsweise Nitrile, wie Acetonitril und Propionitril, ferner Amide, wie Dimethylformamid, Dimethylacetamid und Hexamethylphosphorsäuretriamid, und außerdem Nitromethan, Dimethylsulfoxid, Tetramethylensulfon und N-Methylpyrrolidon.

Die Reaktionstemperaturen können in einem größeren Bereich variiert werden. Im allgemeinen arbeitet man bei Temperaturen zwischen 20 °C und 200 °C, vorzugsweise zwischen 50 °C und 140 °C.

Der Druck ist bei der Durchführung des erfindungsgemäßen Verfahrens nicht wesentlich. Im

allgemeinen arbeitet man bei Normaldruck. Es ist jedoch auch möglich, bei geringfügig erhöhtem oder vermindertem Druck, zum Beispiel zwischen 0,5 und 5 bar, zu arbeiten.

Bei der Durchführung des erfindungsgemäßen Verfahrens setzt man im allgemeinen auf 1 Mol an Halogenverbindung der Formel (II) bis zu 2,5 Mol, vorzugsweise 1,2 bis 2,0 Mol an Dihydroxybenzol der Formel (III) sowie 0,8 bis 1,5 Mol vorzugsweise 1,0 bis 1,2 Mol an Calciumhydroxid ein.

Im allgemeinen arbeitet man bei dem erfindungsgemäßen Verfahren in der Weise, daß man das Dihydroxybenzol der Formel (III) zusammen mit dem Calciumhydroxid im Solvens dispergiert vorlegt und dann die Halogenverbindung der Formel (II) in Substanz oder in Lösung hinzugibt, wobei man die Reaktionstemperatur gegebenenfalls schon vor der Zugabe der Verbindung der Formel (II) einstellt. Es ist aber auch möglich, sämtliche Reaktionspartner bei Raumtemperatur, das heißt zwischen etwa 10 °C und 30 °C, im Solvens zu dispergieren und danach die komplette Mischung auf die Reaktionstemperatur zu bringen.

Die Aufarbeitung erfolgt nach üblichen Methoden. Im allgemeinen verfährt man so, daß man das Reaktionsgemisch nach beendeter Umsetzung in Wasser gießt, mit Salzsäure oder Schwefelsäure ansäuert und das Produkt abtrennt. In der Regel fallen die Reaktionsprodukte nach dem Ansäuren kristallin an und können filtriert werden.

Die nach dem erfindungsgemäßen Verfahren herstellbaren Hydroxyphenyläther der Formel (I) sind wertvolle Ausgangsstoffe zur Synthese von Aryloxycarbonsäure — Derivaten, welche hervorragende herbizide Eigenschaften besitzen (vgl. Belgische Patentschrift 853 574).

So läßt sich beispeilsweise der 3- (2-Nitro-4-Trifluormethyl-phenoxy)-α-phenoxypropionsäure-methylester der Formel

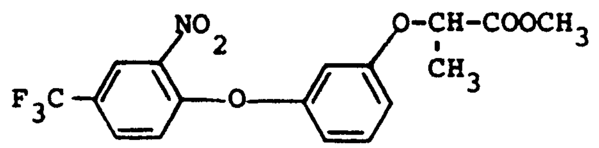

herstellen, indem man 2-Nitro-4-trifluormethyl-3'-hydroxy-diphenyläther in Gegenwart von Methanol und Natriummethylat mit α-Brompropionsäure-methylester umsetzt*. Diese Synthese läßt sich formelmäßig wie folgt wiedergeben:

* Es ist auch möglich, in Gegenwart von Acetonitril und Kaliumcarbonat zu arbeiten.

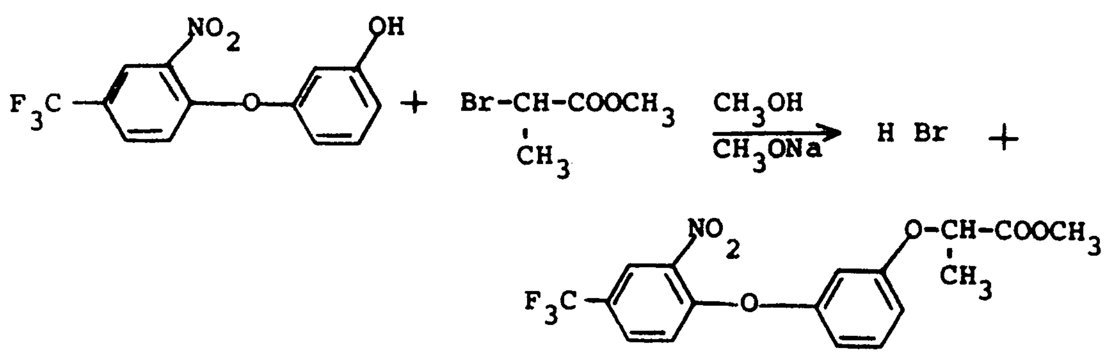


Das erfindungsgemäße Verfahren wird durch die nachfolgenden Beispiele veranschaulicht :

Beispiel 1

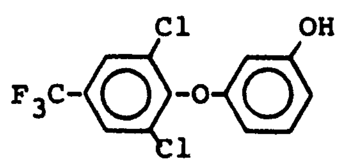

a) Herstellung nach dem erfindungsgemäßen Verfahren :

In eine Mischung aus 330 g (3 Mol) Resorcin und 111 g (1,5 mol) Calciumhydroxid in 2 l Dimethylsulfoxid werden bei 120-130 °C innerhalb von 7 Stunden 358 g (1,5 Mol) 3,4,5-Trichlorbenzotrifluorid getropft. Die Reaktionsmischung wird danach 8 Stunden bei 130 °C nachgerührt. Nach Abkühlung auf Raumtemperatur wird das Reaktionsgemisch in 7 l Wasser gegossen, wobei sich das Reaktionsprodukt ölig abscheidet. Man extrahiert mit 3 l Toluol, trennt die organische Phase ab und engt nach dem Trocknen ein. Der verbleibende Rückstand wird destilliert. Man erhält auf diese Weise 350 g (73 % der Theorie) an 2,6-Dichlor-4-trifluormethyl-3'-hydroxy-diphenyläther in Form eines Festproduktes vom

Schmelzpunkt 64-65 °C.

Sdp. 123-130 °C/0,15 mm Hg

Analyse :

Summenformel : $C_{13}H_7Cl_2F_3O_2$

Berechnet : 48,3 % C ; 2,2 % H ; 22,0 % Cl

Gefunden : 48,3 % C ; 2,3 % H ; 21,8 % Cl

b) Herstellung nach bekannten Verfahren unter Verwendung von Natriumhydroxid als Base :

In eine Mischung aus 330 g (3 Mol) Resorcin und 60 g (1,5 Mol) Natriumhydroxid in 2 l Dimethylsulfoxid werden bei 120-130 °C innerhalb von 7 Stunden 358 g (1,5 Mol) 3,4,5-Trichlor-benzotrifluorid eingetropft. Die Reaktionsmischung wird danach 8 Stunden bei 130 °C gerührt. Nach Abkühlung auf Raumtemperatur wird das Reaktionsgemisch in eine Lösung von 80 g Natriumhydroxid in 7 l Wasser eingerührt. Der ungelöste Anteil wird abgetrennt und in Toluol aufgenommen ; gemäß Dünnschichtchromatogramm enthält diese Lösung keinen 2,6-Dichlor-4-trifluormethyl-3'-hydroxi-diphenyläther. Zur Aufarbeitung der wäßrigen Phase säuert man diese mit Salzsäure an und extrahiert das sich abscheidende Öl mit Toluol. Nach dem Trocknen wird das Lösungsmittel abgezogen und der verbleibende Rückstand destilliert. Man erhält auf diese Weise 30 g (6,7 % der Theorie) an 2,6-Dichlor-4-trifluormethyl-3'-hydroxy-diphenyläther.

Sdp. 123-130 °C/0,15 mm Hg

Beispiel 2

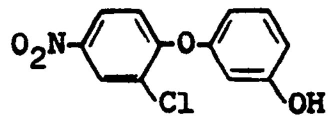

Eine Lösung von 192 g (1 Mol) 1,2-Dichlor-4-nitrobenzol in 300 ml Dimethylsulfoxid wird unter Rühren zu einer Mischung aus 220 g (2 Mol) Resorcin, 74 g (1 Mol) Calciumhydroxid und 1 l Dimethylsulfoxid gegeben. Man erwärmt 5 Stunden unter Rühren auf 80-85 °C. Danach wird das Reaktionsgemisch auf Raumtemperatur abgekühlt und in wässrige Salzsäure gegossen, wobei das Produkt kristallin ausfällt. Nach Absaugen erhält man 220,6 g (83 % der Theorie) 3-Hydroxy-2'-chlor-4'-nitro-diphenyläther in Form gelber Kristalle vom Schmelzpunkt 94-95 °C.

In gleicher Weise werden die in der nachstehenden Tabelle 1 aufgeführten Verbindungen der Formel

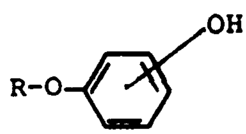

(I)

hergestellt.

Tabelle 1

| Beispiel Nr. | R | Stellung der OH-Gruppe | Produkt | Ausbeute (% der Theorie) | Schmelzpunkt (°C) |
|---|---|---|---|---|---|
| 3 | 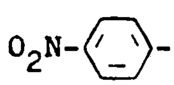 | 3 | 1-Hydroxy-3-(4-nitro-phenoxy)-benzol | 70 | 93 |
| 4 | 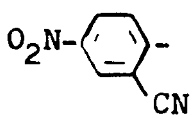 | 3 | 1-Hydroxy-3-(2-cyano-4-nitro-phenoxy)-benzol | 71 | 145 |
| 5 | 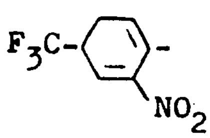 | 3 | 1-Hydroxy-3-(2-nitro-4-trifluor-methyl-phenoxy)-benzol | 79 | (Öl) |
| 6 | 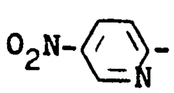 | 3 | 1-Hydroxy-3-(5-nitropyridin(2)yl-oxy)-benzol | 85 | 114 |

Tabelle 1 (Fortsetzung)

| Beispiel Nr. | R | Stellung der OH-Gruppe | Produkt | Ausbeute (% der Theorie) | Schmelz-punkt (°C) |
|---|---|---|---|---|---|
| 7 | 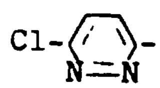 | 3 | 1-Hydroxy-3-(6-chlor-pyridazin(3)yl-oxy)-benzol | 71 | 189 |
| 8 | 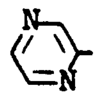 | 3 | 1-Hydroxy-3-pyrazin(2)yl-oxy)-benzol | 72 | 101 |
| 9 | 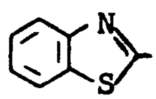 | 3 | 1-Hydroxy-3-benzthiazol(2)yl-oxy)-benzol | 71 | 146 |
| 10 | 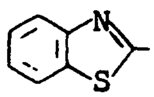 | 4 | 1-Hydroxy-4-benzthiazol(2)yl-oxy)-benzol | 81 | 169 |
| 11 | 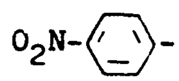 | 4 | 1-Hydroxy-4-(4-nitrophenoxy)-benzol | 98 | 171 |
| 12 | 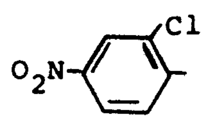 | 4 | 1-Hydroxy-4-(2-chlor-4-nitrophenoxy)-benzol | 98 | 149 |
| 13 | 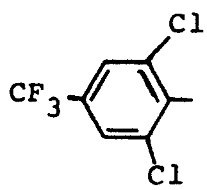 | 4 | 1-Hydroxy-4-(2,6-dichlor-4-trifluor-methyl-phenoxy)-benzol | 78 | 136 |

Herstellung von 3-(2-Nitro-4-trifluormethyl-phenoxy)-α-phenoxy-propionsäure-methylester ausgehend von 2-Nitro-4-trifluormethyl-3'-hydroxy-diphenyläther

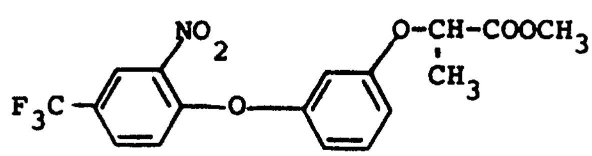

17 g (0,057 Mol) 2-Nitro-4-trifluormethyl-3'-hydroxydiphenyläther und 9,1 g Kaliumcarbonat werden 1 Stunde in 80 ml Acetonitril unter Rückfluß gekocht. Dann tropft man bei 50-55 °C innerhalb von 1 Stunde 11,4 g 2-Brompropionsäuremethylester hinzu und rührt noch 6 Stunden bei 50-55 °C nach.

Zur Aufarbeitung wird das Reaktionsgemisch in 300 ml Wasser gegossen und mit 500 ml Toluol extrahiert. Das Toluol wird dann in Vakuum abdestilliert. Man erhält auf diese Weise 18,5 g (84,3 % der Therorie) an 3-(2-Nitro-4-trifluormethyl-phenoxy)-α-phenoxy-propionsäure-methylester in Form eines gelblichen Öles.

Analyse :
Summenformel : $C_{17}H_{14}F_3NO_6$
Berechnet : 53 % C ; 3,6 % H ; 3,6 % N
Gefunden : 52,8 % C ; 3,5 % H ; 3,5 % N

## Ansprüche

1. Verfahren zur Herstellung von Hydroxyphenyläthern, dadurch gekennzeichnet, daß man Halogen-Verbindungen der Formel

R—Hal (II)

in welcher R für einen Rest der Formel

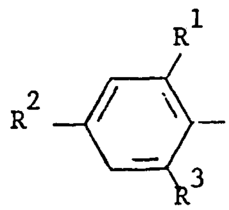

steht,

worin $R^1$, $R^2$ und $R^3$ gleichartig oder verschieden sind und für Wasserstoff, Chlor, Trifluormethyl, Nitro oder Cyano stehen, wobei jedoch mindestens einer der Reste $R^1$, $R^2$ und $R^3$ für Trifluormethyl, Nitro oder Cyano steht und mindestens einer der Reste $R^1$, $R^2$ und $R^3$ für Wasserstoff steht, und

R ferner für einen gegebenenfalls durch Chlor, Nitro und oder Cyano substituierten 5-oder 6-gliedrigen heterocyclischen Rest steht, der 1-3 Heteroatome, wie Stickstoff, Schwefel und/oder Sauerstoff im Ring enthalten kann und außerdem mit einem gegebenenfalls durch Chlor und/oder Trifluormethyl substituierten Benzolring anelliert sein kann, und Hal für Chlor oder Brom steht, mit Dihydroxybenzolen der Formel

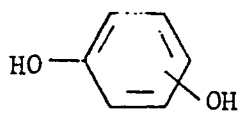

(III)

in Gegenwart von Calciumhydroxid sowie in Gegenwart eines polaren Verdünnungsmittels bei Temperaturen zwischen 20 °C und 200 °C umsetzt.

2. Verfahren gemäß Anspruch 1, dadurch gekennzeichnet, daß man die Umsetzung bei Temperaturen zwischen 50 °C und 140 °C durchführt.

3. Verfahren gemäß Anspruch 1, dadurch gekennzeichnet, daß man die Umsetzung in Gegenwart von Acetonitril, Propionitril, Dimethylformamid, Dimethylacetamid, Hexamethylphosphorsäuretriamid, Nitromethan, Dimethylsulfoxid, Tetramethylensulfon oder N-Methylpyrrolidon durchführt.

4. Verfahren gemäß Anspruch 1, dadurch gekennzeichnet, daß man auf 1 Mol an Halogenverbindung der Formel (II) bis zu 2,5 Mol an Dihydroxybenzol der Formel (III) sowie 0,8 bis 1,5 Mol an Calciumhydroxid einsetzt.

**Claims**

1. Process for the preparation of hydroxyphenyl ethers, characterised in that halogen compounds of the formula

R—Hal (II)

in which R represents a radical of the formula

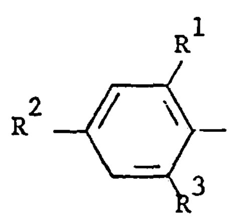

wherein $R^1$, $R^2$ and $R^3$ are identical or different and represent hydrogen, chlorine, trifluoromethyl, nitro or cyano ; however, at least one of the radicals $R^1$, $R^2$ and $R^3$ represents trifluoromethyl, nitro or cyano and at least one of the radicals $R^1$, $R^2$ and $R^3$ represents hydrogen, and

R furthermore represents a 5- or 6-membered heterocyclic radical which is optionally substituted by chlorine, nitro and/or cyano and which can contain in the ring 1-3 hetero atoms such as nitrogen, sulphur and/or oxygen and which can additionally be fused with a benzene ring which is optionally substituted by

chlorine and/or trifluoromethyl, and Hal represents chlorine or bromine, are reacted with dihydroxybenzenes of the formula

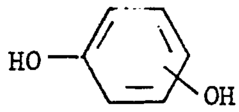

(III)

in the presence of calcium hydroxide and in the presence of a polar diluent, at temperatures between 20 °C and 200 °C.

2. Process according to Claim 1, characterised in that the reaction is carried out at temperatures between 50 °C and 140 °C.

3. Process according to Claim 1, characterised in that the reaction is carried out in the presence of acetonitrile, propionitrile, dimethylformamide, dimethylacetamide, hexamethylphosphoric acid triamide, nitromethane, dimethylsulphoxide, tetramethylene sulphone or N-methylpyrrolidone.

4. Process according to Claim 1, characterised in that up to 2.5 mols of dihydroxybenzene of the formula (III) and 0.8 to 1.5 mols of calcium hydroxide are employed per 1 mol of halogen compound of the formula (II).

**Revendications**

1. Procédé de préparation d'éthers hydroxyphényliques, caractérisé en ce que l'on fait réagir des composés halogénés répondant à la formule

R—Hal (II)

dans laquelle R représente un reste de formule

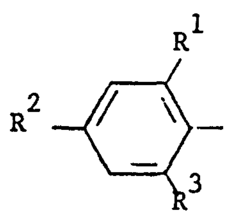

dans laquelle $R^1$, $R^2$ et $R^3$, identiques ou différents, représentent chacun l'hydrogène, le chlore, un groupe trifluorométhyle, nitro ou cyano, l'un au moins des symboles $R^1$, $R^2$ et $R^3$ représentant un groupe trifluorométhyle, nitro ou cyano et l'un au moins des symboles $R^1$, $R^2$ et $R^3$ représentant l'hydrogène,

R pouvant en outre représenter un reste hétérocyclique à 5 ou 6 chaînons éventuellement substitué par le chlore, des groupes nitro et/ou cyano, qui contient de 1 à 3 hétéroatomes comme l'azote, le soufre et/ou l'oxygène dans le noyau, et peut en outre être condensé avec un noyau benzénique éventuellement substitué par le chlore et/ou des groupes trifluorométhyle, Hal représentant le chlore ou le brome, avec des dihydroxybenzènes répondant à la formule

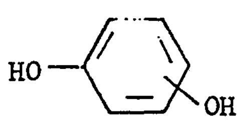

(III)

en présence d'hydroxyde de calcium et en présence d'un diluant polaire à des températures de 20 à 200 °C.

2. Procédé selon la revendication 1, caractérisé en ce que la réaction est effectuée à des températures de 50 à 140 °C.

3. Procédé selon la revendication 1, caractérisé en ce que la réaction est effectuée en présence d'acétonitrile, de propionitrile, de diméthylformamide, de diméthylacétamide, d'hexaméthylphosphorotriamide, de nitrométhane, de diméthylsulfoxyde, de tétraméthylènesulfone ou de N-méthylpyrrolidone.

4. Procédé selon la revendication 1, caractérisé en ce que, pour 1 mole du composé halogéné de formule II, on utilise jusqu'à 2,5 moles du dihydroxybenzène de formule III et de 0,8 à 1,5 mole d'hydroxyde de calcium.